# NEW EUROPEAN PATENT SPECIFICATION

(11) **EP 1 671 609 B2**
(45) Date of publication and mention of the opposition decision: **09.07.2014**
(45) Mention of the grant of the patent: 10.08.2011
(21) Application number: 04029983.6
(22) Date of filing: 17.12.2004
(51) Int. Cl.: A61F 13/511

(54) **Discontinuous lotion application onto the topsheet of an absorbent article**
Unregelmässiger Auftrag einer Lotion auf die obere Lage eines absorbierenden Artikels
Application discontinue d'une lotion sur la couche supérieure d'un article absorbant

(43) Date of publication of application: 21.06.2006
(73) Proprietor: THE PROCTER & GAMBLE COMPANY, Cincinnati, OH 45202 (US)
(72) Inventor: Virgilio, Raffaele, 65824 Schwalbach am Taunus (DE); Borbach, Markus, 65929 Frankfurt (DE); Dziezok, Peter, 65239 Hochheim (DE); Geilich, Ralf, 74654 Crailsheim (DE); Blanco, Agustin Ramos, 61440 Oberursel (DE); Schmidt, Mattias, 65510 Idstein (DE)
(74) Representative: Heide, Ute

(56) References cited:
- WO-A-02/22104
- US-A1- 2001 025 162
- US-A1- 2003 114 812

## Description

### Description

### Field of the Invention

The present invention relates to absorbent articles such as disposable diapers, sanitary napkins and panty liners. More specifically, the present invention relates to topsheets of such articles, i.e. cover layers to be brought in contact with the wearer. According to the present invention a lotion is applied to such topsheets in a specific manner.

### Background of the Invention

Disposable absorbent articles are broadly available and consumers are used to a high performance for the collecting and retaining of menses (in the case of sanitary napkins or panty liners) or for the collecting and retaining urine and fecal material (in the case of e.g. disposable diapers). From such articles consumers expect a superior absorbency behavior and at the same time expect excellent wearing comfort and dryness when being worn.

Often, such articles comprise multiple absorbent members, at least one member being primarily designed to store liquid, and at least one other member primarily designed to acquire and / or distribute liquid, the members typically being encapsulated between a topsheet (on the wearer facing side) and a backsheet (on the garment facing side).

In order to increase the wearing comfort of the absorbent article it is known to apply a lotion coating to those portions of the absorbent article which will come into contact with the wearer, namely the topsheet. The lotion may provide multiple skin care benefits, for example prevent diaper rash. Further, the lotion may have the function of preventing bowel movements from adhering to the wearer's skin.

Such lotions are disclosed for example in US 3 585 998, which discloses a disposable baby diaper havIng an interior liner carrying an away of pressure ruptureable capsules containing baby oil.

US 3 489 148 discloses a baby diaper comprising a hydrophobic and oliophobic top sheet wherein a portion of the topsheet is coated with a film of oleaginous material. While the film, on a micro scale, is described as being discontinuous, a macroscopically uniform film area covers a large portion of the topsheet. Other areas of the topsheet are not covered at all.

US 6 426 444 discloses the application of lotion to a topsheet in the form of a plurality of stripes that are separated by a plurality of stripes having no lotion.

US 2002/0128615 discloses non-aqueous compositions for absorbent articles with can be applied to the bodyside liner of the article in the form of multiple stripes. Patterns including from 1 to 20 stripes of composition extending along the longitudinal direction are disclosed. For the disclosed patterns a lotion stripe covers the longitudinal centreline.

As to provide the desired absorbency to the article, at least the storage member will often comprise super-absorbent material, which is admixed with the traditionally used pulp fiber material. Such super-absorbent materials can absorb many times (e.g.10, 20 or 30 times) their own weight and are therefore very helpful when designing an article of improved fluid handling properties. Many recent products employ higher and higher concentrations of super-absorbent materials, namely concentrations in excess of 50% of the total weight of the storage member. These products achieve a high absorbing capacity with a very thin storage member and are thereby typically overall thin products. While super-absorbent materials can store very large amounts of liquid, they are often not able to distribute the liquid from the point of impact to more remote areas of the absorbent article and to acquire the liquid as fast as it may be received by the article.
WO 02/22104 A2 discloses a composite material including a substrate with first and a second surface, a boundary layer which is comprised of one or more compositions, and a topical application. The boundary layer is applied on the first surface of the substrate, and the topical application is applied to a surface of the boundary layer opposite of the substrate.

The provision of a lotion is normally not beneficial for the absorbency behaviour of the absorbent article. In many cases, in fact, a lotioned absorbent article will provide lesser absorbency and a less good liquid distribution than the same article not provided with the lotion. In one aspect, a lotion provided in liquid form may be partially absorbed by the article, thereby being of no use to the wearer and further decreasing the absorbent capacity of the article. In another aspect, some lotions are hydrophobic and will tend to block the liquid distribution within any layer of the article, in which the lotion is present.

Therefore, it is difficult for the skilled person to provide a lotion absorbent article, which on the one hand delivers lotion in a form ensuring a high wearing comfort and on the other hand has excellent absorbency and dryness attributes. Achieving both is even more difficult in a scenario of low cost mass production. However, as absorbent articles are most typically disposable products it is also important to allow for low cost mass production of these articles. In view of all this, the present invention addresses the following objectives:

It is one objective of the present invention to provide an absorbent article, which has improved liquid handling characteristics as compared to the above disclosed articles.

Moreover, it is an objective of the present invention to provide an article, which is more comfortable to wear, and which in particular provides superior dryness.

In one further important aspect it is an objective of the present invention to provide an absorbent article comprising a lotion which contributes to the wearing comfort of the article, for example by providing skin care benefits.

In yet a further aspect it is an objective of the present invention to provide an absorbent article which is suitable for low cost mass production.

### Summary of the invention

The present invention relates to absorbent articles as defined in claim 1, such as diapers and sanitary napkins, and topsheets useful for such articles.

### Brief description of the drawings

While the specification concludes with claims pointing out and distinctly claiming the present invention, it is believed the same will be better understood by the following drawings taken in conjunction with the accompanying specification wherein like components are given the same reference number.
Figure 1 is a top plan view of a disposable diaper, with the upper layers partially cut away.
Figure 2 is a cross-sectional view of the disposable diaper shown in Figure 1.

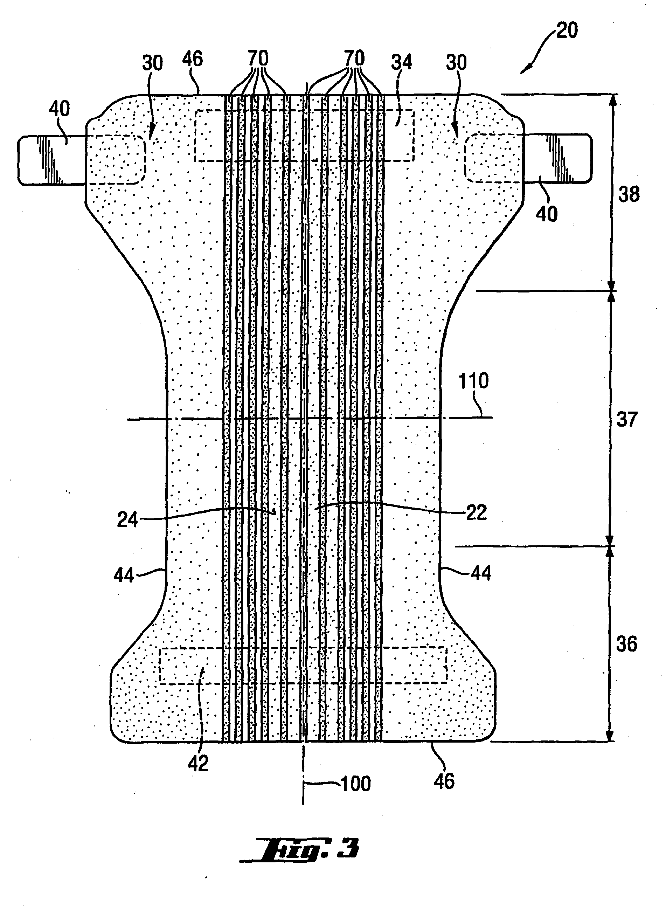

Figure 3 is a top plan view of a disposable diaper as shown in Figure 1, in which complete layers are shown, but the leg cuffs are not shown, and a lotion pattern outside the scope of the present invention is represented.

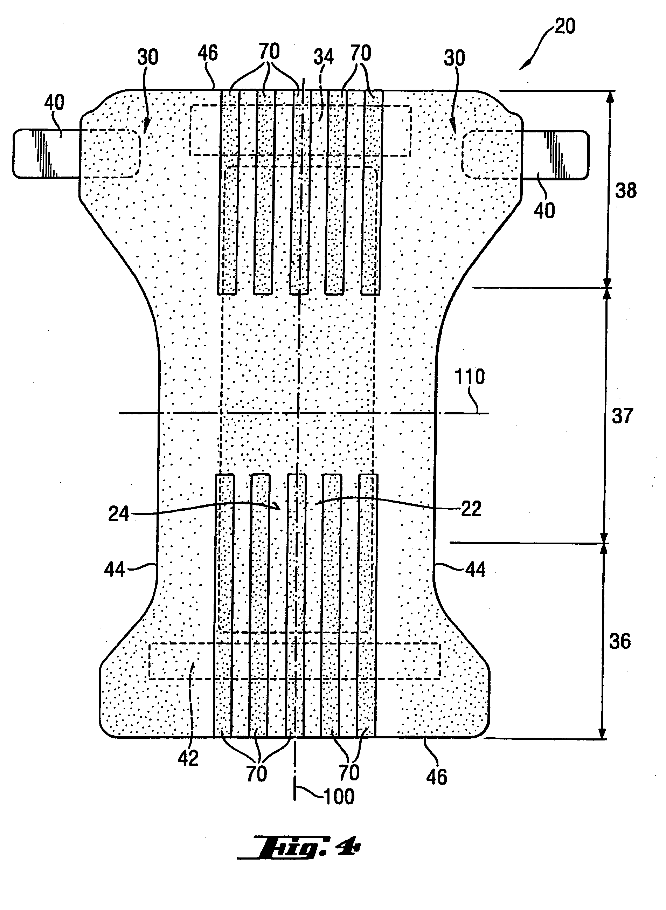

Figure 4 is a top plan view as shown in Figure 3, in which another preferred lotion pattern is represented.
Figure 5 is a top plan view as shown in Figure 3, in which another lotion pattern outside the scope of the present invention is represented.

### Detailed description of the invention

### Definitions

As used herein, the following terms have the following meanings:

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinent briefs, training pants, diaper holders and liners, sanitary napkins and the like. Absorbent articles also include wipes, such as household cleaning wipes, baby wipes, and the like.

"Disposable" is used herein to describe articles that are generally not intended to be laundered or otherwise restored or reused i.e., they are intended to be discarded after a single use and, preferably, to be recycled, composted or otherwise disposed of in an environmentally compatible manner.

"Disposed" is used to mean that an element(s) is formed (joined and positioned) in a particular place or position as a unitary structure with other elements or as a separate element joined to another element.

"Diaper" refers to an absorbent article generally worn by infants and incontinent persons about the lower torso.

The terms "thickness" and "caliper" are used herein interchangeably.

"Attached" or "Joined" encompasses configurations whereby an element is directly secured to another element by affixing the element directly to the other element, and configurations whereby an element is indirectly secured to another element by affixing the element to intermediate member(s) which in turn are affixed to the other element.

"Comprise," "comprising," and "comprises" is an open ended term that specifies the presence of what follows e.g. a component but does not preclude the presents of other features, elements, steps or components known in the art, or disclosed herein.

The term "hydrophilic" describes fibers or surfaces of fibers, which are wettable by aqueous fluids (e.g. aqueous body fluids) deposited on these fibers. Hydrophilicity and wettability are typically defined in terms of contact angle and the strike through time of the fluids, for example through a nonwoven fabric. This is discussed in detail in the American Chemical Society publication entitled "Contact angle, wettability and adhesion", edited by Robert F. Gould (Copyright 1964). A fiber or surface of a fiber is said to be wetted by a fluid (i.e. hydrophilic) when either the contact angle between the fluid and the fiber, or its surface, is less than 90°, or when the fluid tends to spread spontaneously across the surface of the fiber, both conditions are normally co-existing. Conversely, a fiber or surface of the fiber is considered to be hydrophobic if the contact angle is greater than 90° and the fluid does not spread spontaneously across the surface of the fiber.

The terms "fiber" and "filament" are used interchangeably.

The terms "nonwoven", "nonwoven fabric" and "nonwoven web" are used interchangeable.

The disposable article 20 has two centerlines, a longitudinal centerline L and a transverse centerline T.

The term "longitudinal", as used herein, refers to a line, axis or direction in the plane of the disposable article 20 that is generally aligned with (e.g., approximately parallel to) a vertical plane which bisects a standing wearer into left and right body halves when the disposable article 20 is worn.

The terms "transverse" or "lateral" as used herein, are interchangeable, and refer to a line, axis or direction which lies in the plane of the disposable article 20 that is generally perpendicular to the longitudinal direction.

The term discontinuous is to be understood as follows: A continuous stripe pattern of lotion stripes is a pattern having stripes extending in one direction over the full length of the area defined by the outmost boundaries of the stripe pattern (for example: extending over the full length in longitudinal direction) and comprising stripes of equal width (in the example: to be measured in the transversal direction) and being separated by lotion free areas of equal width (in the example: to be measured in the transversal direction). As used herein, a pattern of a plurality of stripes is a discontinuous pattern, when either the stripes are not of equal width or the lotion free areas separating the stripes are not of equal width or the stripes do not extend over the full length of the area defined by the outmost boundaries of the stripe pattern or a combination thereof is present.

### Absorbent articles

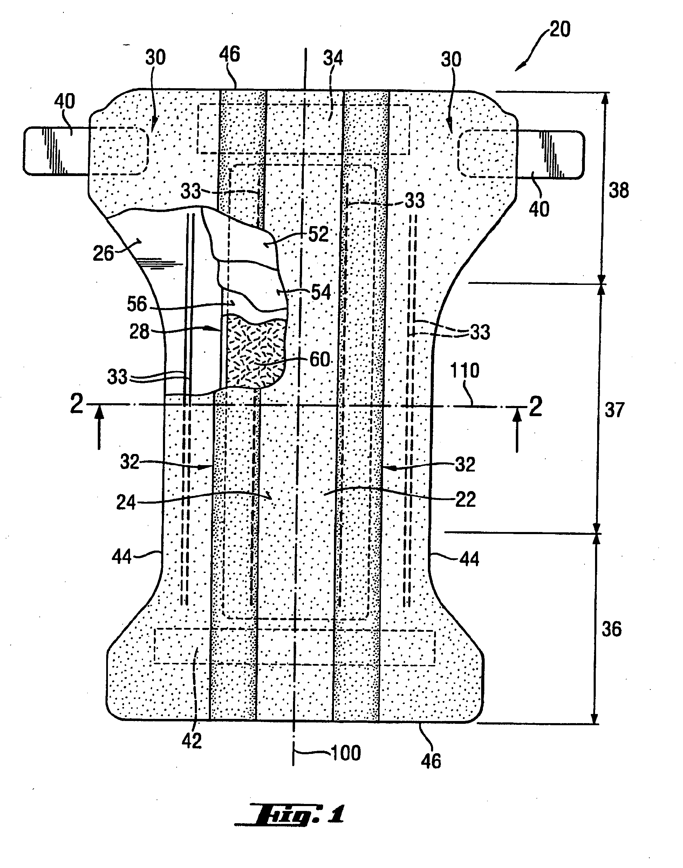

Figure 1 is a plan view of a diaper 20 as a preferred embodiment of an absorbent article according to the present invention. The diaper is shown in its flat out, uncontracted state (i.e., without elastic induced contraction). Portions of the structure are cut away to more clearly show the underlying structure of the diaper 20. The portion of the diaper 20 that contacts a wearer is facing the viewer. The chassis 22 of the diaper 20 in Figure 1 comprises the main body of the diaper 20. The chassis 22 comprises an outer covering including a liquid pervious topsheet 24 and/or a liquid impervious backsheet 26. The chassis may also include most or all of the absorbent core 28 encased between the topsheet 24 and the backsheet 26. The chassis preferably further includes side panels 30, leg cuffs 32 and a waist feature 34. The leg cuffs and the waist feature typically comprise elastic members 33. One end portion of the diaper 20 is configured as the front waist region 36 of the diaper 20. The opposite end portion is configured as the rear waist region 38 of the diaper 20. An intermediate portion of the diaper 20 is configured as the crotch region 37, which extends longitudinally between the front and rear waist regions 36 and 38. The crotch region 37 is that portion of the diaper 20 which, when the diaper 20 is worn, is generally positioned between the wearer's legs. The waist regions 36 and 38 may include a fastening system comprising fastening members 40 preferably attached to the rear waist region 38 and a landing zone 42 attached to the front waist region 36. The diaper 20 has a longitudinal axis 100 and a transverse axis 110. The periphery of the diaper 20 is defined by the outer edges of the diaper 20 in which the longitudinal edges 44 run generally parallel to the longitudinal axis 100 of the diaper 20 and the end edges 46 run generally parallel to the transverse axis 110 of the diaper 20.

For unitary absorbent articles, the chassis 22 comprises the main structure of the diaper with otherfeatures added to form the composite diaper structure. While the topsheet 24, the backsheet 26, and the absorbent core 28 may be assembled in a variety of well-known configurations, preferred diaper configurations are described generally in U.S. Pat. No. 5,569,234 entitled "Disposable Pull-On Pant" issued to Buell et al. on October 29, 1996; and U.S. Patent No. 6,004,306 entitled "Absorbent Article With MultiDirectional Extensible Side Panels" issued to Robles et al. on December 21, 1999.

The topsheet 24 in Figure 1 may be fully or partially elasticized or may be foreshortened to provide a void space between the topsheet 24 and the absorbent core 28. Exemplary structures including elasticized or foreshortened topsheets are described in more detail in U.S. Pat. No. 5,037,416 entitled "Disposable Absorbent Article Having Elastically Extensible Topsheet" issued to Allen et al. on August 6, 1991; and U.S. Pat. No. 5,269,775 entitled "Trisection Topsheets for Disposable Absorbent Articles and Disposable Absorbent Articles Having Such Trisection Topsheets" issued to Freeland et al. on December 14, 1993.

The backsheet 26 in Figure 1 is generally the portion of the diaper 20 positioned with the absorbent core 28 between the backsheet 26 and the topsheet 24. The backsheet 26 may be joined with the topsheet 24. The backsheet 26 prevents the exudates absorbed by the absorbent core 28 and contained within the article 20 from soiling other external articles that may contact the diaper 20, such as bed sheets and undergarments. In preferred embodiments, the backsheet 26 is substantially imperviousto liquids (e.g., urine) and comprises a laminate of a nonwoven and a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962, and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation

### EXXAIRE.

The absorbent core 28 in Figure 1 generally is disposed between the topsheet 24 and the backsheet 26. The absorbent core 28 may comprise any absorbent material that is generally compressible, conformable, non-irritating to the wearer's skin, and capable of absorbing and retaining liquids such as urine and other certain body exudates. The absorbent core 28 may comprise a wide variety of liquid-absorbent materials commonly used in disposable diapers and other absorbent articles such as comminuted wood pulp, which is generally referred to as air left. Examples of other suitable absorbent materials include creped cellulose wadding; melt blown polymers, including co-form; chemically stiffened, modified or cross-linked cellulosic fibers; tissue, including tissue wraps and tissue laminates, absorbent foams, absorbent sponges, superabsorbent polymers, absorbent gelling materials, or any other known absorbent material or combinations of materials. The absorbent core may further comprise minor amounts (typically less than 10%) of non-liquid absorbent materials, such as adhesives, waxes, oils and the like.

Exemplary absorbent structures for use as the absorbent assemblies are described in U.S. Patent 4,834,735, entitled "High Density Absorbent Members Having Lower Density and Lower Basis Weight Acquisition Zones", issued to Alernany et al. on May 30, 1989; and U.S. Patent No. 5,625,222 entitled "Absorbent Foam Materials For Aqueous Fluids Made From high Internal Phase Emulsions Having Very High Water-To-Oil Ratios" Issued to DesMarais et al. on July 22, 1997.

The diaper 20 may also include such other features as are known in the art including front and rear ear panels, waist cap features, elastics and the like to provide better fit, containment and aesthetic characteristics. Such additional features are well known in the art and are described in U.S. Pat. No. 3,860,003 entitled "Contractable side portions for disposable diaper" issued to Buell et al. on January 14, 1975 and U.S. Patent No. 5,151,092 entitled "Absorbent article with dynamic elastic waist feature having a predisposed resilient flexural hinge" issued to Buell et al. on September 29, 1992.

In order to keep the diaper 20 in place about the wearer, the waist regions 36 and 38 may include a fastening system comprising fastening members 40 preferably attached to the rear waist region 38. In a preferred embodiment the fastening system further comprises a landing zone 42 attached to the front waist region 36. The fastening member is attached to the front waist region 36, preferably to the landing zone 42 to form leg openings and an article waist.

Diapers 20 according to the present invention may be provided with a re-closable fastening system or may alternatively be provided in the form of pant-type diapers.

The fastening system and any component thereof may include any material suitable for such a use, including but not limited to plastics, films, foams, nonwoven webs, woven webs, paper, laminates, fiber reinforced plastics and the like, or combinations thereof. It may be preferable that the materials making up the fastening device be flexible. The flexibility is designed to allow the fastening system to conform to the shape of the body and thus, reduces the likelihood that the fastening system will irritate or injure the wearer's skin.

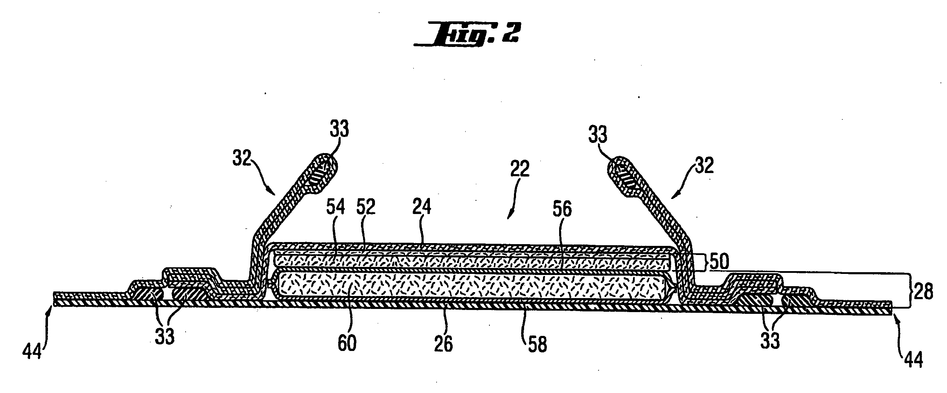

Figure 2 shows a cross-sectional view of Figure 1 taken In the transverse axis 110. Starting from the wearer facing side the diaper comprises the topsheet 24, the components of the absorbent core 28, and the backsheet 26. The absorbent core preferably comprises an acquisition system 50, which comprises an upper acquisition layer 52 facing towards the wearer and a lower acquisition layer 54. In one preferred embodiment the upper acquisition layer comprises a nonwoven fabric whereas the lower acquisition layer preferably comprises a mixture of chemically stiffened, twisted and curled fibers, high surface area fibers and thermoplastic binding fibers. In another preferred embodiment both acquisition layers are provided from a non-woven material, which is preferably hydrophilic. The acquisition layer preferably is In direct contact with the storage layer 60.

The storage layer 60 is preferably wrapped by a core wrap material. In one preferred embodiment the core wrap material comprises a top layer 56 and a bottom layer 58. The top layer 56 and the bottom layer 58 can be provided from a non-woven material. One preferred material is a so-called SMS material, comprising a spunbonded, a melt-blown and a further spunbonded layers. The top layer 56 and the bottom layer 58 may be provided from two or more separate sheets of materials or they may be alternatively provided from a unitary sheet of material. Such a unitary sheet of material may be wrapped around the storage layer 60, e.g. in a C-fold. The top layer 56 and the bottom layer 58 may also be joined to each other, preferably along their periphery. In one preferred option both layers are joined along their longitudinal peripheries, in other embodiments they are joined along the transversal peripheries, or along the longitudinal and the transversal peripheries. The joining can be achieved my multiple means well known in the art, eg. by adhesive means, using a continuous or a discontinuous pattern, and preferably a linear or curvilinear pattern.

The storage layer 60 typically comprises fibrous materials, mixed with superabsorbent, absorbent gelling materials. Other materials described above as suitable for the absorbent core 28 may also be comprised. Storage layer according to the present invention may comprise a superabsorbent material in an amount corresponding to at least 90% of the total weight of the storage layer.

A upper liquid acquisition layer 52 useful in a diaper according to the present invention may comprise any of the nonwoven fabrics described below. A preferred liquid acquisition layer 52 comprises a binder, the binder preferably comprising a styrene-butadiene latex binder. Preferably, the styrene-butadiene latex binder has a carboxylation level of at least 10 %, preferably at least 12 %. Preferably, the upper liquid acquisition layer 52 comprises polyester fibers and the liquid acquisition layer comprises 20 to 40 weight percent of styrene-butadiene latex binder, and 60 to 80 weight percent of said polyester fibers. Even more preferably, the polyester fibers comprise 20 to 80 weight percent of a first type of fibers, and 20 to 80 weight percent of a second type of fibers, the second type of fibers comprising spiral-crimp fibers. Highly preferred are upper liquid acquisition layers wherein the first type of fibers exhibits a flat crimp and wherein the second type of fibers comprises hollow chemically homogeneous bi-component fibers. Also highly preferred are any upper liquid acquisition layers wherein the polyester fibers are carded to form a nonwoven.

Preferred acquisition systems may also comprise superabsorbent materials. Such acquisition systems may also comprise a single acquisition layer or multiple acquisition layers. Where multiple acquisition layers are comprised any of these layer may comprise super-absorbent materials. Such superabsorbent material may be comprised in an amount corresponding to at least 30%, at least 40%, at least 50%, at least 60%, at least 70%, at least 80% or at least 90%, at times even in an amount of 100 % of the total weight of the respective acquisition layer.

### Nonwoven fabrics

A nonwoven fabric is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled.

The fibers may be of natural or man-made origin. They may be staple or continuous filaments or be formed in situ.

Nonwoven fabrics can be formed by many processes such as meltblowing, spunbonding, carding. The basis weight of nonwoven fabrics is usually expressed in grams per square meter (gsm).

Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Fibers are classified according to their origin, chemical structure, orboth. They can be braided into ropes and cordage, made into felts (also called nonwovens or non-woven fabrics), woven or knitted into textile fabrics, or, in the case of high-strength fibers, used as reinforcements in composites—that is, products made of two or more different materials.

The nonwoven fabrics may comprise fibers made by nature (natural fibers), made by man (synthetic orman-made), or combinations thereof. Example natural fibers include but are not limited to: animal fibers such as wool, silk, fur, and hair; vegetable fibers such as cellulose, cotton, flax, linen, and hemp; and certain naturally occurring mineral fibers. Synthetic fibers can be derived from natural fibers ornot. Examplesyntheticfibers, which are derived from natural fibers include but are not limited to rayon and lyocell, both of which are derived from cellulose, a natural polysaccharide fiber. Synthetic fibers, which are not derived from natural fibers can be derived from other natural sources or from mineral sources. Example synthetic fibers not derived from natural sources include but are not limited to polysaccharides such as starch. Example fibers from mineral sources include but are not limited to polyolefin fibers such as polypropylene, polyethylene fibers and polyester, which are derived from petroleum, and silicate fibers such as glass and asbestos.

Nonwoven webs can be formed by direct extrusion processes during which the fibers and webs are formed at about the same point in time, or by preformed fibers, which can be laid into webs at a distinctly subsequent point in time. Example direct extrusion processes include but are not limited to: spunbonding, meltblowing, solvent spinning, electro-spinning, and combinations thereof typically forming layers.

Example "laying" processes include wetlaying and drylaying. Example drylaying processes include but are not limited to airlaying, carding, and combinations thereof typically forming layers. Combinations of the above processes yield nonwovens commonly called hybrids or composites. Example combinations include but are not limited to spunbond-meltblown-spunbond (SMS), spunbond-carded (SC), spunbond-airlaid (SA), melt-blown-airlaid (MA), and combinations thereof, typically in layers. Combinations which include direct extrusion can be combined at about the same point in time as the direct extrusion process (e.g., spinform and coform for SA and MA), or at a subsequent point in time. In the above examples, one or more individual layers can be created by each process. For instance, SMS can mean a three layer, 'sms' web, a five layer 'ssmms' web, or any reasonable variation thereof wherein the lower case letters designate individual layers and the uppercase letters designate the compilation of similar, adjacent layers.

The fibers in a nonwoven web are typically joined to one or more adjacent fibers at some of the overlapping junctions. This includes joining fibers within each layer and joining fibers between layers when there is more than one layer. Fibers can be joined by mechanical entanglement, by chemical bond or by combinations thereof. Fibers can also be joined by heat-bonding, which comprises techniques such as through-air bonding and thermobonding by use of heated calendar rolls.

### Preferred topsheets

All of the above described fibers and manufacturing techniques can be useful for providing topsheets according to the present invention.

The topsheet of the diaper can be provided from a hydrophilic material to promote rapid transfer of liquids (e.g., urine) through the topsheet. If the topsheet is made of a hydrophobic material, at least the upper surface of the topsheet is preferably treated to be hydrophilic so that liquids will transfer through the topsheet more rapidly. It can use be preferable to provide a hydrophobic material with large pores which allow for sufficient liquid transport. Any such topsheet embodiments diminish the likelihood that body exudates will flow off the topsheet rather than being drawn through the topsheet and being absorbed by the absorbent core. The topsheet can be rendered hydrophilic by treating it with a surfactant. Suitable methods for treating the topsheet with a surfactant include spraying the topsheet material with the surfactant and immersing the material into the surfactant.

Lotion compositions useful for the present invention comprise: (1) an emollient(s); (2) an immobilizing agent(s) for the emollient; (3) optionally a hydrophilic surfactant(s); and (4) other optional components.

The viscosity of the such lotion compositions, including emollient, immobilizing agent, and optional components should be as high as possible to keep the lotion from flowing into the interior of the article. Unfortunately, high viscosities can also lead to lotion compositions that are difficult to apply without processing problems. Therefore, a balance must be achieved so the viscosities are high enough to keep the lotion compositions localized on the surface of the article topsheet, but not so high as to cause processing problems. Suitable viscosities for the lotion compositions will typically range from about 5 to about 200 centipoises, preferably from about 15 to about 100 centipoises, measured at 60° C.

The lotion compositions preferably are solid, or more often semisolid, at 20° C., i.e. at ambient temperatures. By "semisolid" is meant that the lotion composition has a rheology typical of pseudoplastic or plastic fluids. When no shear is applied, the lotion compositions can have the appearance of a semi-solid but can be made to flow as the shear rate is increased. This is due to the fact that, while the lotion composition contains primarily solid components, it also includes some minor liquid components.

The article topsheets of the present invention contain an effective amount of the lotion composition. As used herein, the term "effective amount of a lotion coating" refers to an amount of a particular lotion composition which, when applied to a article topsheet, will be effective for promoting the skin health of the wearer or in some circumstances effective for reducing the adherence of BM to the skin of the wearer. Of course, the effective amount of a lotion coating will depend, to a large extent, on the particular lotion composition used.

Preferably 0.02 g to 2 g, more preferably 0.04 g to 0.4 g, yet more preferably 0.08 to 0.2 g of lotion are applied to the topsheet of the absorbent article. Preferably this is achieved by applying 2 to 200 g per square meter (more preferably 5 to 50 g per square meter, yet more preferably 10 to 20 g per square meter) of lotion within the lotioned area (lotion stripes) of the topsheet.

For the application of lotion compositions in accordance with the present invention multiple techniques are suitable, namely spraying, gravure coating and extrusion coating methods are preferred. All of these methods are known to the skilled person.

According to one conventional approach known in this area, the lotion has been applied relatively uniformly over a large area of the topsheets, using any of the above methods, often spraying. As some lotions, in particular hydrophobic lotions, are known to hinder the absorption of liquids, at least when they are used in high concentrations, some of these relatively uniform patterns have employed lotion free windows. Preferably, the lotion free windows have been disposed in the crotch area of the absorbent articles.

According to the alternative approach, the lotion has been applied to the topsheet in a stripe pattern. Specifically, US 6 426 444 has disclosed the application in stripes (70) of uniform width, typically from 0.1 inch to 1 inch (0.254 cm to 2.54 cm). The stripes (70) are arranged in a uniform pattern, i.e. all stripes (70) have the same width and all stripes (70) are separated by stripes having no lotion, which in turn also have the same width. Such a stripe pattern is preferable over patterns in which relatively large areas are uniformly filled with lotion, as many lotion free areas are provided and as the stripe pattern represents a very cost effective method of application.

However, when conceiving the present invention it has been found that the stripe pattern can have some disadvantages, in particular when a topsheet comprising a stripe pattern is combined with an acquisition system comprising relatively high concentrations of super-absorbent material. When, for example, the stripes (70) are oriented in the longitudinal direction, it has been found that the distribution of liquid in the lateral direction is not satisfactory. Without wishing to be bound by any theory, it seems that the liquid is predominantly distributed along the longitudinal direction while distribution in the lateral direction is relatively poor. This leads to an incomplete usage of the storage capacity of the storage layer. It has been found that the problem is particularly acute for a uniform stripe pattern as such pattern can almost completely block the lateral distribution of liquid. This leads to significantly reduced acquisition times and thereby to relatively poor absorbency and hence potentially to a sensation of wetness by the wearer.

The above described problems may also occur when the acquisition system is free of superabsorbent material, but the storage layer comprises high amounts of superabsorbent material, for example more than 50% of superabsorbent material. Again a continuous stripe pattern can lead to very poor liquid distribution in one direction. A continuous stripe pattern along the longitudinal directon has been found to very significantly reduce liquid distribution along the transversal direction. Also if superabsorbent material is present only in the storage layer this leads to slow and poor acquisition of liquid and may therefore also lead to a need of providing an increased amount of super-absorbent materials, making the disposable article undesirably expensive.

When conceiving the present invention, it has been surprisingly found, that all of the objectives stated above can be met by providing a discontinuous stripe pattern. Hitherto it has been known to either apply lotion to a large and continuous area (e.g. by spraying) while providing lotion free areas elsewhere, orto apply lotioned and lotion free area in combination using stripes (e.g. by roll-on application) such stripe pattern, however, extending uniformly over essentially the whole topsheet area. Hence, conceptually different lotion application patterns known and so far only considered as being alternatives have been combined now. On the one hand, the easy to apply stripe pattern is used, but on the other hand a relatively large area is left free of lotion.

In one highly preferred embodiment of the present invention longitudinal stripes (70) are employed. In a first zone stripes (70) of relatively small distance are employed, i.e. the lotion free zones between the stripes (70) have a relatively small width. Preferably, the lotion free zones have a width from 1 mm to 10 mm, more preferably from 1 mm to 5 mm. Transversally adjacent to the narrow stripe zone there is a second zone of stripes (70) having a larger distance. In this area the lotion free stripes (70) preferably have a distance from 5 mm to 40 mm, more preferably from 20 mm to 40 mm, yet more preferably 30 mm to 40 mm. Adjacent to this zone another, third small distance stripe zone is used, for which the same distances are preferred as for the first zone described above. The second zone may also comprise only a lotion free area, which provides a discontinuity to the stripe pattern provided by the first and third zone. Preferably, one lotion free stripe of the second zone comprises the transverse, and most preferably also the longitudinal centreline of the absorbent article.

Another preferred embodiment also employs longitudinal stripes. These stripes (70) are separated by lotion free stripes (70) in the longitudinal direction which are of equal distance. In this pattern a discontinuity is created by interrupting at least one lotion stripe in the longitudinal direction. Preferably the lotion stripes (70) are interrupted over a length of 10 mm to 100 mm, more preferably 30 mm to 70 mm, yet more preferably 40 mm to 60 mm. The area defined by these interruption comprises the transverse centreline of the disposable article.

In other preferred embodiments of the present invention the plurality of stripes (70) is oriented parallel with the transverse centerline. The pattern as described above the longitudinal stripes can also be used for transverse stripes, the distances along the transverse direction given above are then to be measured along the longitudinal direction and *vice versa.*

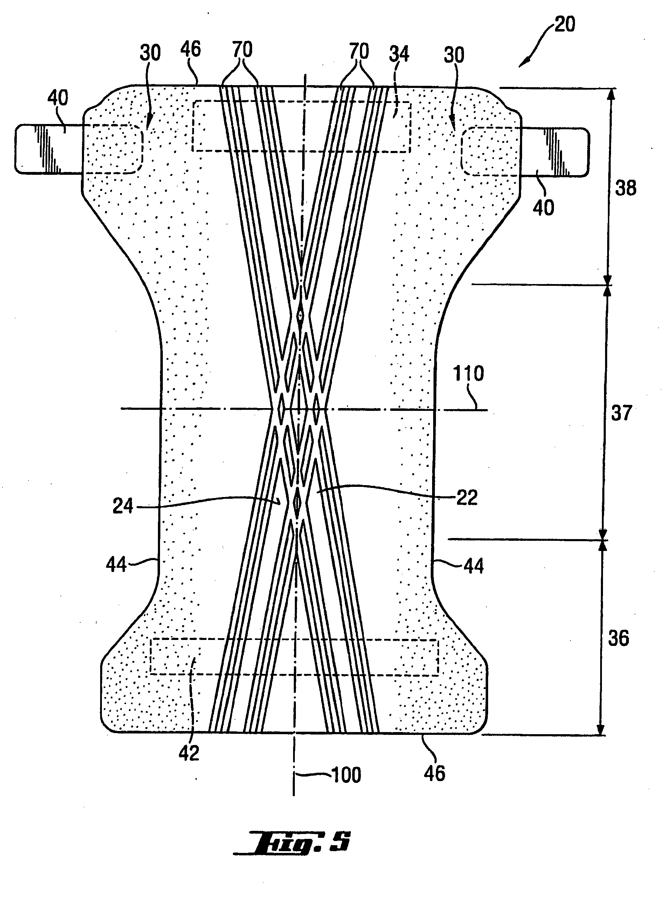

In yet further embodiments the pattern comprises two pluralities of discontinuous stripes, the pluralities not being parallel to each other as shown in Fig. 5

For any stripe pattern according to the present invention the pattern comprises a lotion-free area which comprises the transverse centerline of the disposable article or the transverse centerline and the longitudinal centerline.

## Claims

1. An absorbent article (20) comprising: - a liquid impervious backsheet (26);
- a liquid pervious topsheet (24) joined to said backsheet (26), said topsheet (24) having an inner surface oriented toward an interior of said article (20) and an outer surface oriented toward the skin of a wearer when said article is being worn, wherein at least a portion of said topsheet outer surface comprises a lotion
- an absorbent core (28) positioned between said topsheet (24) and said backsheet (26);
- wherein a lotion is applied to the liquid pervious topsheet (24) in the form of a pattern of a plurality of stripes (70), that are separated by a plurality of areas having no lotion; the disposable absorbent article (20) being **characterized in that** the pattern of a plurality of stripes (70) is a discontinuous pattern, and wherein the absorbent core comprises a storage member comprising super-absorbent material in an amount corresponding to at least 90% of the total weight of the storage layer,
wherein said pattern comprises a lotion-free area which comprises the transverse centerline of the disposable article (20).

2. The disposable article (20) of claim 1 wherein the topsheet is hydrophobic and comprises pores.

3. The disposable article (20) of claim 1 wherein the topsheet comprises a hydrophobic material treated to expose a hydrophilic surface.

4. The disposable article (20) of any one of the preceding claims wherein the lotion is partially transferable to the wearer's skin.

5. The disposable article (20) of any one of the preceding claims wherein the lotion coating is semi-solid or solid at 20°C.

6. The disposable article (20) of claim 1 wherein the plurality of striped (70) is oriented parallel with the longitudinal centerline.

7. The disposable article (20) of any one of the preceding claims wherein the absorbent article comprises an acquisition system, the acquisition system comprising super-absorbent material.

8. The disposable article (20) of any one of the preceding claims wherein the acquisition system comprises super-absorbent material in a concentration of in excess of 50% of the total weight of the acquisition system.

## Patentansprüche

1. Absorptionsartikel (20), umfassend:
- eine flüssigkeitsundurchlässige Unterschicht (26),
- eine flüssigkeitsdurchlässige Oberschicht (24), die mit der Unterschicht (26) verbunden ist, wobei die Oberschicht (24) eine Innenoberfläche, die zum Inneren des Artikels (20) ausgerichtet ist, und eine Außenoberfläche, die zur Haut eines Trägers ausgerichtet ist, wenn der Artikel getragen wird, aufweist, wobei mindestens ein Abschnitt der Außenoberfläche der Oberschicht eine Lotion umfasst,
- einen Absorptionskern (28), der zwischen der Oberschicht (24) und der Unterschicht (26) angeordnet ist,
- wobei eine Lotion in der Form eines Musters mehrerer Streifen (70), die durch mehrere Bereiche ohne Lotion getrennt sind, auf die flüssigkeitsdurchlässige Oberschicht (24) aufgetragen ist,
wobei der Einwegabsorptionsartikel (20) **dadurch gekennzeichnet ist, dass**
das Muster mehrerer Streifen (70) ein diskontinuierliches Muster ist und wobei der Absorptionskern ein Speicherelement umfasst, das Superabsorbermaterial in einer Menge, die mindestens 90 % des Gesamtgewichts der Speicherschicht entspricht, umfasst,
wobei das Muster einen lotionsfreien Bereich umfasst, der die Querachse des Einwegartikels (20) umfasst.

2. Einwegartikel (20) nach Anspruch 1, wobei die Oberschicht hydrophob ist und Poren umfasst.

3. Einwegartikel (20) nach Anspruch 1, wobei die Oberschicht ein hydrophobes Material umfasst, das behandelt ist, um eine hydrophile Oberfläche freizulegen.

4. Einwegartikel (20) nach einem der vorstehenden Ansprüche, wobei die Lotion teilweise auf die Haut des Trägers übergehen kann.

5. Einwegartikel (20) nach einem der vorstehenden Ansprüche, wobei die Lotionsbeschichtung bei 20 °C halbfest oder fest ist.

6. Einwegartikel (20) nach Anspruch 1, wobei die mehreren Streifen (70) parallel zur Längsachse ausgerichtet sind.

7. Einwegartikel (20) nach einem der vorstehenden Ansprüche, wobei der Absorptionsartikel ein Aufnahmesystem umfasst, wobei das Aufnahmesystem Superabsorbermaterial umfasst.

8. Einwegartikel (20) nach einem der vorstehenden Ansprüche, wobei das Aufnahmesystem Superabsorbermaterial in einer Konzentration von über 50 % des Gesamtgewichts des Aufnahmesystems umfasst.

## Revendications

1. Article absorbant (20) comprenant : - une feuille de fond imperméable aux liquides (26) ;
- une feuille de dessus perméable aux liquides (24) jointe à ladite feuille de fond (26), ladite feuille de dessus (24) ayant une surface interne orientée vers l'intérieur dudit article (20) et une surface externe orientée en direction de la peau d'un porteur lorsque ledit article est porté, dans lequel au moins une partie de ladite surface externe de la feuille de dessus comprend une lotion
- une âme absorbante (28) positionnée entre ladite feuille de dessus (24) et ladite feuille de fond (26) ;
- dans lequel une lotion est appliquée à la feuille de dessus perméable aux liquides (24) sous la forme d'un motif d'une pluralité de bandes (70), qui sont séparées par une pluralité de zones n'ayant aucune lotion ;
l'article absorbant jetable (20) étant **caractérisé en ce que**
le motif d'une pluralité de bandes (70) est un motif discontinu, et dans lequel l'âme absorbante comprend un élément de stockage comprenant un matériau superabsorbant en une quantité correspondant à au moins 90 % du poids total de la couche de stockage, dans lequel ledit motif comprend une zone dépourvue de lotion qui comprend la ligne médiane transversale de l'article jetable (20).

2. Article jetable (20) selon la revendication 1, dans lequel la feuille de dessus est hydrophobe et comprend des pores.

3. Article jetable (20) selon la revendication 1, dans lequel la feuille de dessus comprend un matériau hydrophobe traité pour exposer une surface hydrophile.

4. Article jetable (20) selon l'une quelconque des revendications précédentes, dans lequel la lotion est partiellement transférable sur la peau du porteur.

5. Article jetable (20) selon l'une quelconque des revendications précédentes, dans lequel le revêtement de lotion est semi-solide ou solide à 20 °C.

6. Article jetable (20) selon la revendication 1, dans lequel la pluralité de bandes (70) est orientée parallèle à la ligne médiane longitudinale.

7. Article jetable (20) selon l'une quelconque des revendications précédentes, où l'article absorbant comprend un système de recueil, le système de recueil comprenant un matériau superabsorbant.

8. Article jetable (20) selon l'une quelconque des revendications précédentes, dans lequel le système de recueil comprend un matériau superabsorbant en une concentration de plus de 50 % du poids total du système de recueil.
